# EUROPÄISCHE PATENTANMELDUNG

(11) **EP 1 293 558 A2**
(43) Veröffentlichungstag der Anmeldung: **19.03.2003**
(21) Anmeldenummer: 02019751.3
(22) Anmeldetag: 03.09.2002
(51) Int. Cl.: C12M 1/00, C12M 1/36, C12M 3/00

(54) **Gehäuse für einen Brutschrank mit thermisch abschliessbarem Innenbehälter sowie Verfahren zur Herstellung**

(30) Priorität: 14.09.2001 DE 10145459
(71) Anmelder: KENDRO Laboratory Products GmbH, 63450 Hanau (DE)
(72) Erfinder: Hessler, Egon, 63594 Hasselroth (DE)
(74) Vertreter: Lang, Friedrich, Dipl.-Ing.

(57) **Zusammenfassung**

Ein Gehäuse für einen Begasungs-Brutschrank weist einen gegenüber der äußeren Atmosphäre thermisch abschließbaren Innenbehälter auf, dessen Innenraum über eine Fronttür des Gehäuses zugänglich ist; der Innenbehälter ist wenigstens teilweise von einer thermisch isolierenden Umhüllung aus wenigstens vier plattenförmigen, wärmeisolierenden Elementen umgeben, die in ihrem Kantenbereich jeweils miteinander verbunden sind und den Innenbehälter mantelförmig umgeben; vorteilhafterweise ist die Rückseite der Umhüllung im Bereich der Rückwand des Brutschrankes ebenfalls mit einem plattenförmigen, wärmeisolierenden Element geschlossen. Die plattenförmigen Elemente bestehen aus einem Werkstoff geringer Wärmeleitfähigkeit und sind im Abstand zur Innenseite des äußeren Gehäuses angeordnet. So entsteht zwischen dem Innenbehälter und dem äußeren Gehäuse ein mit Luft gefüllter Innenraum, der durch die Umhüllung in zwei thermisch gegeneinander abgeschlossene Räume mit jeweils eigener Konvektion aufgeteilt ist. Die plattenförmigen Elemente bestehen dabei aus einem schaumförmigen thermoplastischen Kunststoff, welcher Polysterol und Polyphenylenether aufweist.

## Beschreibung

Die Erfindung betrifft ein Gehäuse für einen Brutschrank, insbesondere Begasungs-Brutschrank, mit einem gegenüber der äußeren Atmosphäre thermisch abschließbaren Innenbehälter, dessen Innenraum über eine Fronttür des Gehäuses zugänglich ist, wobei der Innenbehälter wenigstens teilweise von einer Umhüllung aus wenigstens einem Werkstoff geringer Wärmeleitfähigkeit im Abstand umgeben ist, sowie ein Verfahren zur Bildung einer thermisch isolierenden Umhüllung des Innenbehälters.

Aus der DE 38 15 528 ist ein Begasungs-Brutschrank zum Kultivieren von menschlichen oder tierischen Zellen oder Geweben bekannt, mit einem durch eine Tür verschließbaren Innengehäuse, das von einem wärmeisolierenden äußeren Gehäuse umgeben ist. Das äußere Gehäuse weist Seitenwände, eine Rückwand sowie Decke und Boden auf und ist auf seiner Innenseite mit einem freitragenden Wärmeisolationskörper ausgekleidet, wobei zwischen dem Innengehäuse und dem Wärmeisolationskörper ein Zwischenraum zumindest im Bereich der Seitenwände und der Oberseite entsteht.

Als problematisch erweist sich die verhältnismäßig aufwendige Auskleidung des äußeren Gehäuses mit dem Wärmeisolationskörper auf der Innenseite, wobei zuvor passend zugeschnittene Einzelteile für die Auskleidung vorgesehen sind.

Aufgabe der Erfindung ist es, eine preisgünstige Wärmeisolierung für einen thermisch abschließbaren Innenbehälter zu entwickeln, welcher auch bei Temperaturen im Bereich von 90 ― 100 °C - wie sie beim Sterilisieren auftreten - seine Formstabilität behält.

Die Aufgabe wird dadurch gelöst, dass die Umhüllung wenigstens vier plattenförmige, wärmeisolierende Elemente aufweist, die jeweils miteinander verbunden sind und den Innenbehälter mantelförmig umgeben, wobei die plattenförmigen Elemente im Abstand zur Innenseite des äußeren Gehäuses angeordnet sind.

Als besonders vorteilhaft erweist es sich, dass die thermisch isolierende Umhüllung aus plattenförmigen, wärmeisolierenden Elementen so ausgebildet ist, dass sie auch bei erhöhten Temperaturen - beispielsweise bei Sterilisationsvorgängen im Temperaturbereich von 100°C - formstabil bleibt.

Vorteilhafte Ausgestaltungen der Erfindung sind in den Ansprüchen 2 bis 6 angegeben.

In einer vorteilhaften Ausgestaltung nach Anspruch 1 ist die Umhüllung auf ihrer zur Frontseite bzw. zu der für die Fronttür vorgesehenen Seite offen, während sie auf ihrer der Fronttür gegenüberliegenden Rückseite ein zusätzliches plattenförmiges Element aufweist.

Die plattenförmigen Elemente sind jeweils im Kantenbereich miteinander verbunden.

In einer vorteilhaften Ausgestaltung der Erfindung sind die plattenförmigen Elemente jeweils durch Nuten bzw. Vertiefungen sowie nockenartige Erhöhungen im Kantenbereich, die in Vertiefungen eines benachbarten plattenförmigen Elements greifen, formschlüssig miteinander zu verbinden.

Die plattenförmigen Elemente bestehen vorzugsweise aus einem schaumförmigen thermoplastischen Kunststoff, welcher Polystyrol und Polyphenylenether aufweist. Zwischen dem thermisch isolierten Innenbehälter und dem äußeren Gehäuse liegt ein mit Luft gefüllter Innenraum vor, der - mit Ausnahme der Frontseite - durch die thermisch isolierende Umhüllung - zumindest teilweise - in zwei thermisch gegeneinander abgeschlossene Räume mit jeweils eigener Konvektionsströmung aufgeteilt ist.

In einer vorteilhaften Ausgestaltung erfolgt die Halterung des Innenbehälters im Frontbereich des Gehäuses, wobei zwecks thermischer Entkopplung zwischen Innenbehälter und äußerem Gehäuse Befestigungsmittel - z.B. Schrauben - aus Kunststoff bzw. Befestigungsmittel mit thermisch isolierender Oberfläche, eingesetzt werden. An seiner kältesten Stelle im Bodenbereich ist der Innenbehälter auch mit dem äußeren Gehäuse durch thermisch leitende Befestigungsmittel - wie z.B. Schrauben - verbunden.

Die Erfindung wird für ein Verfahren zur Bildung einer thermischen Isolierung eines Innenbehälters für einen Brutschrank, bei dem der Innenbehälter über eine Fronttür des Gehäuses zugänglich ist, und wenigstens teilweise von einer thermisch isolierenden Umhüllung aus wenigstens vier plattenförmigen, wärmeisolierenden Elementen umgeben ist, dadurch gelöst, dass ein Werkstoff in Granulatform, welcher expandierendes Polystyrol und Polyethylen aufweist, in eine den plattenförmigen Elementen der Umhüllung entsprechende Form eingeführt und durch Zufuhr von Dampf zu einem thermisch isolierenden plattenförmigen Formteil gepresst wird. Dabei wird zusätzlich zum Dampf auch ein Gas, wie beispielsweise Pentan benötigt, welches vom Hersteller des Werkstoffs dem Granulat beigefügt ist.

Vorzugsweise wird zur Bildung der plattenförmigen Elemente für die Umhüllung ein Kompound aus einer Mischung aus EPS (expandierbares Polystyrol) und PPE in Form eines Granulats eingesetzt, welches von der Firma Deutsche Shell Chemie GmbH, 65727 Eschborn unter dem Namen Caril gekauft werden kann. Die plattenförmigen Elemente werden durch relativ einfache, preisgünstige Werkzeuge hergestellt, in denen Formteile für zweimal Seitenwände, Decke, Boden und Rückwand des äußeren Gehäuses speziell der Geometrie der jeweiligen Verwendung angepasst werden kann.

Die Platten sind bis zur Montage im Gehäuse vorteilhafterweise sehr einfach zu transportieren und zu handhaben, da eine raumintensive Hohlraumbildung erst beim Aufbau des Gehäuses erfolgt.

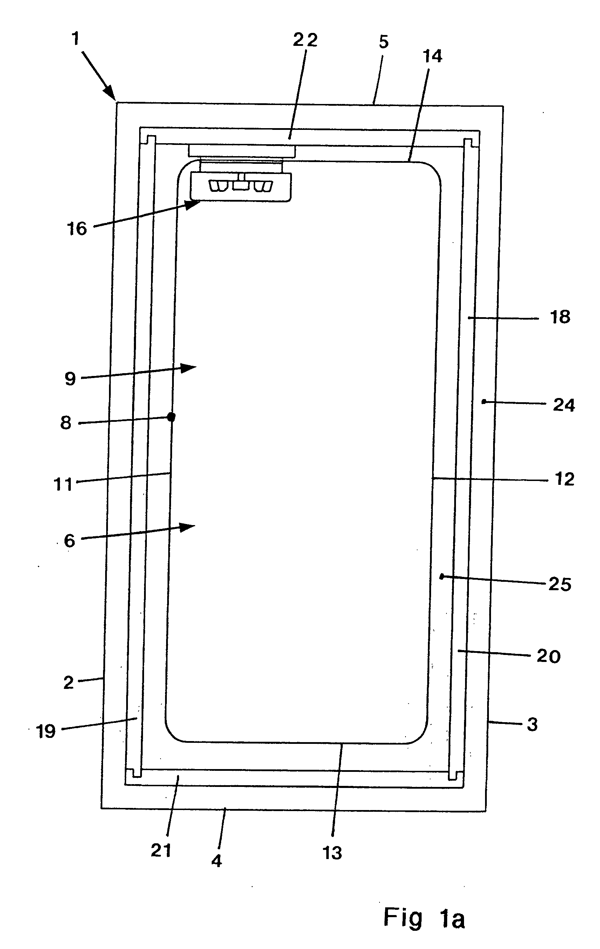

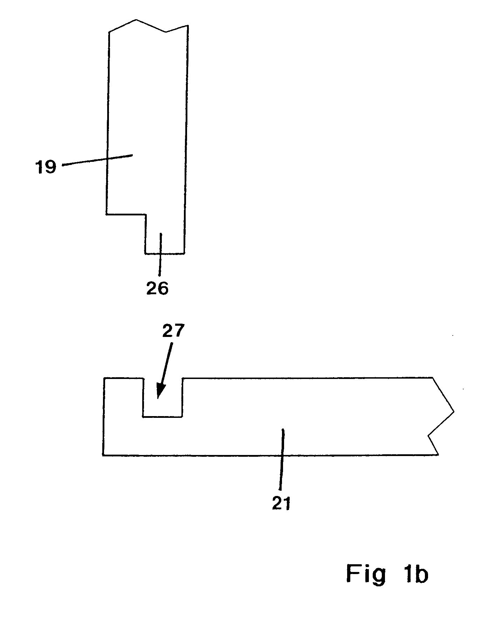

Im folgenden ist der Gegenstand der Erfindung anhand der Figuren 1a, 1 b und 2 näher erläutert.

Figur 1 a zeigt einen Längsschnitt durch ein im Aufbau befindliches Gehäuse für einen Begasungsbrutschrank mit der thermisch isolierenden Umhüllung (von der Rückseite aus gesehen);

Figur 1 b zeigt eine formschlüssige Verbindung im Kantenbereich zwischen zwei wärmeisolierenden plattenförmigen Elementen als Teil der Umhüllung.

Figur 2 zeigt in einer perspektivischen Darstellung den Aufbau der thermisch isolierenden Elemente.

Gemäß Figur 1 a weist das Gehäuse 1 zwei Seitenwände 2, 3, einen Gehäuseboden 4 sowie eine Abdeckung 5 des oberen Gehäuseteils auf. Die Rückwand ist symbolisch mit Bezugsziffer 6 angedeutet.

Im Inneren von Gehäuse 1 befindet sich ein Innenbehälter 8 mit Innenraum 9 als eigentlichem Behandlungsraum des Brutschrankes, wobei der Innenbehälter 8 Seitenwände 11, 12, einen Bodenbereich 13 sowie einen oberen Deckenbereich 14 aufweist. Im Deckenbereich 14 befindet sich eine hier schematisch angedeutete Gaszufuhr für Innenraum 9 zusammen mit einem Ventilator und einer Sensoreinheit, welche symbolisch dargestellt sind und als zusammengehöriger Komplex mit dem Bezugszeichen 16 versehen sind.

Zwischen dem Innenbehälter 8 und dem äußeren Gehäuse 1 ist eine thermisch isolierende Umhüllung 18 mit den beiden seitlichen plattenförmigen Elementen 19, 20, dem plattenförmigen Element 21 im Bodenbereich 21 sowie dem oberen Element 22 im Deckenbereich angeordnet, wobei zwischen Umhüllung 18 und Innenbehälter 8 bzw. zwischen Umhüllung 18 und äußerem Gehäuse 1 jeweils ein Abstand besteht, so dass im Längsschnitt gesehen zwei jeweils getrennte Zwischenräume 24 (außen) und 25 (innen) gebildet werden. Die Zwischenräume 24, 25 sind aufgrund der Umhüllung 18 thermisch gegeneinander isoliert, so dass jeder der beiden Zwischenräume jeweils eine eigene Konvektionsströmung aufweist; vorzugsweise befindet sich in beiden Zwischenräumen 24, 25 Luft. Da der Innenbehälter auch an seiner Rückseite von einem plattenförmigen, wärmeisolierenden Element umgeben ist, sind die beiden Zwischenräume auch im Bereich der hier nicht dargestellten Rückwand des Brutschrankes vorhanden, während die zur Frontseite gerichtete Öffnung des Innenbehälters nicht von der Umhüllung aus plattenförmigen, wärmeisolierenden Elementen umgeben ist.

Anhand Figur 1 b ist schematisch die zu erstellende Verbindung eines gebrochen dargestellten seitlichen Elements 19 der Umhüllung 18 mit einem ebenfalls gebrochen dargestellten Element 21 im Bodenbereich erkennbar, wobei das Zusammenspiel von einem nasenartigen Vorsprung 26 des plattenförmigen Elements 19 und dazu passender Ausnehmung bzw. Nut 27 des plattenförmigen Elements 21 im Bodenbereich erkennbar ist. So weist die Nut 27 im Profil gesehen eine Ausnehmung auf, in die die nasenförmige Erhebung bzw. Vorsprung 26 eingefügt werden kann; zusätzlich ist es möglich, Maßnahmen gegen eine Verschiebung entlang der Längsachse der Nut 27 vorzusehen, beispielsweise durch Abschluss der Nuten im jeweiligen Kantenbereich. Auf diese Weise entstehen trogartige Nuten, die eine Verschiebung des nasenförmigen Vorsprungs 26 des zur Verbindung vorgesehenen benachbarten Elements 19 entlang der Nut 27 nicht mehr zulassen. Weiterhin wird die zur Rückwand 6 des Gehäuses gerichtete Öffnung der Umhüllung (hier nicht gezeigt) durch ein zusätzliches plattenförmiges, wärmeisolierendes Element geschlossen.

Darüber hinaus ist es auch möglich, zwischen zwei benachbarten plattenförmigen Elementen der thermisch isolierenden Umhüllung jeweils eine formschlüssige Verbindung durch Verzinkung im Kantenbereich vorzusehen, wobei schwalbenschwanzartige Zinken in entsprechend geformte Nuten eingreifen.

Bei Einsatz von plattenförmigen thermisch isolierenden Elementen 19, 20, 21, 22 aus einem Kunststoff aus Polymer-Basis ist eine ausreichende Stabilität zur Vormontage der Umhüllung 18 gegeben, so dass diese als kompaktes Bauelement in das Gehäuse 1 eingeführt werden kann.

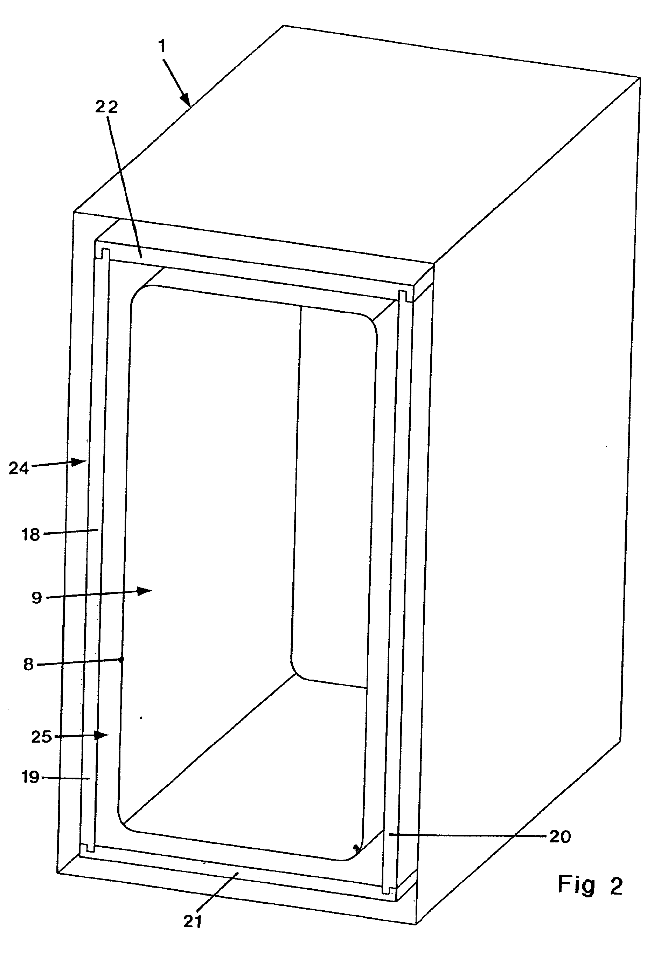

Figur 2 zeigt in einer perspektivischen Ansicht ein im Aufbau befindliches Gehäuse 1, dessen Rückseite noch geöffnet ist, so dass sowohl der Innenraum 9 von Innenbehälter 8 als auch die Umhüllung 18 wenigstens teilweise erkennbar ist.

So ist zwischen der thermisch isolierenden Umhüllung 18 mit ihren Seitenwänden 19, 20 sowie den Elementen 21 im Bodenbereich und Abdeckung 22 im oberen Bereich erkennbar, dass jeweils ein thermisch isolierender Zwischenraum 24 und 25 zum Innenbehälter bzw. zum äußeren Gehäuse vorgesehen ist, welcher für eine intensive thermische Isolation sorgt, wobei die beiden Zwischenräume 24, 25 gegeneinander abgeschlossen sind und jeweils eine eigene thermische Konvektion aufweisen.

Im Zuge der kompletten Montage ist es möglich, den Innenbehälter 8 durch eine Rückwand - beispielsweise aus Metall - abzudichten, welche ggf. mit Wärmeaustauscherelementen (Heizung; Kühlung) versehen sein kann. Weiterhin kann anschließend die thermisch isolierende Umhüllung 18 an der Rückseite des Gehäuses 1 (zur Rückwand 6 gerichtet) ebenfalls mit Hilfe eines zusätzlichen plattenförmigen, thermisch isolierenden Elements geschlossen werden, das hier zwecks besserer Übersicht nicht dargestellt ist.

Der Innenbehälter 8 und das äußere Gehäuse 1 bestehen vorzugsweise aus Metallbiech, wobei der Innenbehälter 8 zwecks verbesserter Wartungs- und Möglichkeiten vorzugsweise aus Edelstahl besteht. Die thermisch isolierende Umhüllung 18 besteht vorzugsweise aus plattenförmigen Elementen, die aus einem Interpolymer aus expandierbarem Polysterol (EPS) und modifiziertem Polyphenyläther (PPE) in Granulatform hergestellt sind. Ein solches Granulat expandierbarer Partikel ist von Shell Chemicals Europe unter der Bezeichnung "Caril" zu erhalten.

Die Herstellung der plattenförmigen thermisch isolierenden Elemente 19, 20, 21, 22 sowie des weiteren plattenförmigen, thermisch isolierenden Elements für die Rückwand erfolgt in der Regel in einem Styropor verarbeitenden Betrieb, wo ein Werkstoff in Granulatform in eine Form gepresst und durch die Zugabe von heißem Wasserdampf expandiert wird, wobei der Werkstoff sich aufgrund des Expansionseffekts in zusammenhängende plattenförmige Teilchen verpressen lässt. Aufgrund von zueinander passenden Nuten bzw. Vertiefungen und nasenförmigen Vorsprüngen bzw. Erhebungen lassen sich die Teile in der Endmontage einfach zusammenstecken; es ist jedoch auch möglich, die Teile durch Zinken bzw. schwalbenschwanzartige formschlüssige Verbindungen miteinander zu verbinden.

Als besonders vorteilhaft erweist sich sowohl der preisgünstige Herstellungsvorgang, der raumsparende Transport, die einfache Vormontage sowie die verhältnismäßig einfache Entsorgung bei Gehäusen - insbesondere Gehäusen für Brutschränke - die zur Verschrottung vorgesehen sind.

## Patentansprüche

1. Gehäuse für einen Brutschrank, insbesondere Begasungs-Brutschrank, mit einem gegenüber der äußeren Atmosphäre thermisch abschließbaren Innenbehälter, dessen Innenraum über eine Fronttür des Gehäuses zugänglich ist, wobei der Innenbehälter wenigstens teilweise von einer thermisch isolierenden Umhüllung aus wenigstens einem Werkstoff geringer Wärmeleitfähigkeit im Abstand umgeben ist, **dadurch gekennzeichnet, dass** die Umhüllung (18) wenigstens vier plattenförmige, wärmeisolierende Elemente (19, 20, 21, 22) aufweist, die jeweils miteinander verbunden sind und den Innenbehälter (8) mantelförmig umgeben, wobei die plattenförmigen Elemente im Abstand zur Innenseite des äußeren Gehäuses (1) angeordnet sind.

2. Gehäuse nach Anspruch 1, **dadurch gekennzeichnet, dass** die thermisch isolierende Umhüllung (18) auf ihrer zur Frontseite des Gehäuses (1) gerichteten Seite offen ist, während sie an ihrer Rückseite zusätzlich ein weiteres plattenförmiges Element aufweist.

3. Gehäuse nach Anspruch 1 oder 2, **dadurch gekennzeichnet, dass** die plattenförmigen Elemente (19, 20, 21, 22) jeweils im Kantenbereich miteinander verbunden sind.

4. Gehäuse nach Anspruch 3, **dadurch gekennzeichnet, dass** die plattenförmigen Elemente (19, 20, 21, 22) jeweils durch Nuten (27) und/oder nasenartige Vorsprünge (26) im Kantenbereich formschlüssig miteinander verbunden sind.

5. Gehäuse nach einem der Ansprüche 1 bis 4, **dadurch gekennzeichnet, dass** die plattenförmigen Elemente (19, 20, 21, 22) aus einem schaumförmigen thermoplastischen Kunststoff bestehen, welcher Polystyrol und Polyphenylenether aufweist.

6. Gehäuse nach einem der Ansprüche 1 bis 5, **dadurch gekennzeichnet, dass** zwischen dem Innenbehälter (8) und dem äußeren Gehäuse (1) ein mit Luft gefüllter Innenraum vorliegt, der durch die Umhüllung (18) zumindest teilweise in zwei thermisch gegeneinander abgeschlossene Räume (24, 25) mit jeweils eigener Konvektion aufgeteilt ist.

7. Verfahren zur Bildung einer thermisch isolierenden Umhüllung des Innenbehälters eines Gehäuses nach einem der Ansprüche 1 bis 6, **dadurch gekennzeichnet, dass** ein Werkstoff in Granulatform, welcher expandierbares Polystyrol und Polyphenylenether aufweist, in eine den plattenförmigen Elementen der thermisch isolierenden Umhüllung des Innenraums entsprechende Form eingeführt und durch Zufuhr von Heißdampf jeweils zu einem thermisch isolierenden plattenförmigen Element gepresst wird.
